**Europäisches Patentamt**

**European Patent Office**

**Office européen des brevets**

(11) Veröffentlichungsnummer: **0 268 224 B1**

# EUROPÄISCHE PATENTSCHRIFT

(45) Veröffentlichungstag der Patentschrift: **29.01.92**

(21) Anmeldenummer: **87116761.5**

(22) Anmeldetag: **13.11.87**

(51) Int. Cl.⁵: **C07C 303/44**, C07C 303/28

(54) **Verfahren zur Isolierung von Alkalisulfat-armen Paraffinsulfonaten und Schwefelsäure aus Paraffin-Sulfoxidation-Reaktionsgemischen ohne Zwangsanfall von Natriumsulfat.**

(30) Priorität: **18.11.86 DE 3639464**

(43) Veröffentlichungstag der Anmeldung:
**25.05.88 Patentblatt 88/21**

(45) Bekanntmachung des Hinweises auf die
Patenterteilung:
**29.01.92 Patentblatt 92/05**

(84) Benannte Vertragsstaaten:
**DE ES FR GB IT NL**

(56) Entgegenhaltungen:
**EP-A- 0 037 883**
**EP-A- 0 143 416**
**DE-A- 2 139 477**

(73) Patentinhaber: **HOECHST AKTIENGESELL-
SCHAFT**
**Postfach 80 03 20**
**W-6230 Frankfurt am Main 80(DE)**

(72) Erfinder: **Pistorius, Rudolf, Dr.**
**Neumühlstrasse 15**
**W-6274 Hünstetten(DE)**

# Beschreibung

Die durch Sulfoxidation von n-Paraffinen z.B. nach dem Verfahren des deutschen Patents 910 165 erhältlichen wässrigen Lösungen von Paraffinsulfonsäuren enthalten außerdem noch Schwefeldioxid, Schwefelsäure und hydrotrop gelöste Paraffine. Um aus solchen Reaktionsgemischen brauchbare Paraffinsulfonsäure bzw. Paraffinsulfonate von gute Qualität, d.h. möglichst schwefelsäure- bzw. salzarme, helle und weitestgehend geruchsfreie Produkte zu isolieren, müssen Schwefeldioxid, Schwefelsäure und Paraffine möglichst quantitativ und schonend abgetrennt werden. Die Paraffinsulfoxidationsprodutke beginn sich bereits bei Temperaturen oberhalb 50 °C zu zersetzen, was sich äußerlich an der Verfärbung des sauren Reaktionsgemisches von wasserhell über gelblich, braun bis schließlich tiefschwarz zeigt. Wenn auch die Menge der durch die Temperatureinwirkung zersetzten Paraffinsulfonsäure noch relativ gering ist, solange die sauren Reaktionsgemische nicht über längere Zeit Temperaturen über 100 °C ausgesetzt sind, erfordert jedoch bereits ein kleiner Anteil an zersetzten Produkten wegen ihrer Farbintensität einen beträchtlichen Bleichaufwand, wenn man zu einwandfrei hellen Produkten kommen will.

Es wurde festgestellt, daß demgegenüber alkalisch reagierende Salze der Paraffinsulfonsäuren relativ stabil sind. Temperaturen unter 200 °C führen auch bei längerer Erhitzungsdauer nur zu ganz unwesentlichen Verfärbungen, die sich noch leicht mit geringen Bleichmittelmengen wieder beseitigen lassen.

Es muß daher bereits beim ersten Schritt der Aufarbeitung der Paraffin-Sulfoxidations-Reaktionsgemische, d.h. bei der Entgasung zur Entfernung des Schwefeldioxids, darauf geachtet werden, daß möglichst keine Verfärbung auftritt. Wird die Entgasung im schwachen Vakuum ausgeführt, so bedarf es nur eines sehr kurzzeitigen Erwärmens auf ca. 85 °C, um eine nahezu vollständige Eliminierung des Schwefeldioxids zu erreichen. Ebenso möglich ist ein Ausblasen mit Inertgas oder auch mit reinem Sauerstoff in einer mit einer geeigneten Packung gefüllten Säule bei einer Temperatur von ca. 40-85 °C.

Durch unmittelbar anschließendes Wiederabkühlen des Reaktionsgemisches auf Raumtemperatur kann bei diesem Prozeßschritt eine merkliche Zersetzung, d.h. eine eintretende Farbvertiefung des Reaktionsgemisches verhindert werden.

Im Hinblick auf die Qualität des Paraffinsulfonats wäre es günstig, das Reaktionsgemisch nach der Entgasung sofort zu neutralisieren. Eine derartige Verfahrensweise ist jedoch wegen des zur Neutralisierung der Schwefelsäure erforderlichen hohen Verbrauchs an Alkali sowie wegen der beachtlichen Verluste an Paraffinsulfonat, die beim Abfiltrieren des Alkalisulfats auftreten, unwirtschaftlich und technisch aufwendig.

Nach der Erfindung des Schwefeldioxids aus dem Reaktionsgemisch muß deshalb versucht werden, vor der Neutralisation die Schwefelsäure möglichst vollständig und unter Schonung der Paraffinsulfonsäure aus dem Gemisch abzutrennen. Bei den bekannten Verfahren, die ein solches Ziel anstreben, geht man im allgemeinen so vor, daß man das entgaste Sulfoxidationsgemisch mit einem geeigneten organischen Lösungsmittel behandelt, um eine Entmischung in eine organische Phase, welche die Paraffinsulfonsäuren enthält und in eine wäßrige Phase, welche die Schwefelsäure soweit wie möglich in Form einer im allgemeinen 10 bis 25 %igen wäßrigen Lösung enthält, herbeizuführen. Die beiden Phasen werden dann getrennt und die organische Phase zur Isolierung der Paraffinsulfonsäuren bzw. ihrer Salze weiter aufgearbeitet. So ist es aus der am 29.1.1953 bekanntgemachten deutschen Patentanmeldung F 3718, 120 bereits bekannt, wasserunlösliche oder nur beschränkt mit Wasser mischbare organische Lösungsmittel, wie z.B. Benzol, Chlorbenzol, Cyclohexan, Tetrachlorkohlenstoff, Chloroform, Methylenchlorid und dergleichen, zur Abtrennung von Schwefelsäure dem Sulfoxidationsgemisch zuzusetzten. Gemäß DE-OS 27 30 245 kommen für den gleichen Zweck auch Ether wie z.B. Diethylether oder Di-n-butylether und gemäß DE-OS 27 45 691 auch Ketone oder Ester sowie gemäß DE-OS 21 39 477 auch Alkohole mit mindestens 5 Kohlenstoffatomen zur Anwendung.

Keines dieser bekannten Verfahren zur Abtrennung von Schwefelsäure bei niederen Temperaturen hat sich bislang großtechnisch durchsetzen können, weil entweder der Aufwand bei der destillativen Aufarbeitung der Produktlösung zu groß und/oder der Abscheidungsgrad der Schwefelsäure aus dem Reaktionsgemisch nicht ausreichte, um schließlich zu salzarmen Produkten zu gelangen, die weniger als 2 Gew.-% Restsalz (bezogen auf 100 % Paraffin-Sulfonat) enthalten.

So kann man z.B. mit Alkoholen mit 4 bis 6 C-Atomen bei einstufiger Extraktion die Schwefelsäure nur so unvollständig abtrennen, daß im neutralisierten Endprodukt der Salzgehalt immer noch wesentlich über 2 Gew.-% (bezogen auf Paraffin-Sulfonat) liegt, auch wenn die zugesetzte Alkoholmenge auf 30 Gew.-% (bezogen auf das entgaste Sulfoxidations-Reaktionsgemisch) gesteigert wird. Größere oder kleinere Alkoholmengen führen dagegen zu einer noch weniger vollständigen Abscheidung der Schwefelsäure.

Gibt man jedoch z.B. nach der Abscheidung mit Hexanol nochmals Wasser hinzu, um weitere Schwefelsäure aus dem Reaktionsgemisch auszuwaschen (2-stufige Extraktion), damit der Restsalzgehalt im Paraffinsulfonat schließlich 2 Gew.-5 (bezogen wiederum auf die waschaktive Substanz) nicht übersteigt, so stellt man fest, daß hierzu nicht unbeträchtliche Wassermengen erforderlich sind, die sich zudem nur zu einem kleineren Teil wieder abscheiden, wodurch der destillative Aufwand sehr stark ansteigt.

Einerseits nimmt der Grad der Schwefelsäureabscheidung mit steigender C-Zahl der eingesetzten Alkohole zu, andererseits wächst aber dadurch der Aufarbeitungsaufwand mit dem Ansteigen der Siedepunkte der verwendeten Alkohole.

In der DE-OS 3 342 984 wird ebenfalls ein Verfahren zur Isolierung von Alkalisulfat-armen Paraffinsulfonaten und Schwefelsäure aus Paraffin-Sulfoxidation-Reaktionsgemischen mit Hilfe von Alkoholen beschrieben.

Ausgangspunkt ist dabei das bei der Sulfoxidation von n-Paraffinen, insbesondere von $C_{13}$-$C_{18}$-Paraffinen, erhaltene, durch Entgasung vom Schwefeldioxide befreite Reaktionsgemisch, das bei Temperaturen von 15 bis 80 °C, insbesondere bis 25 bis 35 °C mit 15 bis 30, insbesondere 17 bis 25 Gew.-% eines $C_4$-$C_8$-Alkohols verrührt wird. Bevorzugt ist hierbei Isobutanol.

Diese Alkansulfonsäure/Alkohol-Lösung wird kontinuierlich in eine Apparatur eingegeben, die aus drei kombinierten Misch-Absetzgefäßen (sogen. Mixer-Settler) und einem weiteren Mischgefäß besteht.

In den 1. Mixer werden die Paraffinsulfonsäure/Alkohol-Lösung, die Oberphase des 2. Settlers und die Unterphase des 3. Settlers eindosiert, in den 2. Mixer die Unterphase des 1. Settlers und soviel von der Alkansulfonsäure/Alkohol-Lösung, daß der pH-Wert im 2. Mixer-Settler immer zwischen 7 und 8 leigt. Der 3. Mixer nimmt die gesamte benötigte Alkalilauge auf und die Produktphase des 1. Settlers. Ausgeschleust werden aus dem 2. Settler die Unterphase, die aus einer wäßrigen Alkalisulfatlösung mit einer Spur Alkohol (unter 1 Gew.-%) besteht, und die obere Phase aus dem 3. Settler, die im Mischgefäß mit weiterer Alkansulfonsäure/Alkohol-Lösung auf den pH-Wert von ca. 11 eingestellt wird. Diese Lösung wird dann bis zu dem gewünschten Konzentrationsgrad eingedampft.

Von gravierenden Nachteil bei dieser Verfahrenweise ist der Zwangsanfall von wäßrigen Alkalisulfatlösungen, die zudem noch mit Spuren des verwendeten Alkohols verunreinigt sind. Wegen der starken Schaumneigung dieser Lösung können diese Akoholspuren kaum destillativ entfernt werden. Bevor ein solches Abwasser der biologischen Kläranlage zugeführt werden kann, muß normalerweise auch das Betonwände zerstörend Sulfat noch abgetrennt werden, was weitere Entsorgungsprobleme nach sich zieht.

Es wurde nun gefunden, daß man diese Nachteile des bekannten Verfahrens vermeiden kann, wenn man ein weiteres Misch-Absetzgefäß einführt und die wässrigen Alkalisulfatlösungen über dieses Gefäß wieder in den Kreislauf einbringt. Gegenstand der Erfindung ist somit ein Verfahren zur Isolierung von Alkalisulfat-armen Alkansulfonaten und Schwefelsäure aus Paraffin-Sulfoxidation-Reaktionsgemischen mit Hilfe von Alkoholen, bei dem man das von Schwefeldioxid befreite Reaktionsgemisch in einem Misch-Absetzgefäß (1) mit einem $C_4$-$C_8$-Alkohol und der Unterphase aus einem Misch-Absetzgefäß (2) vermischt, die sich abscheidende untere Phase aus verdünnter Schwefelsäure abtrennt, die verbleibende obere Produktphase zusammen mit der Unterphase eines Misch-Absetzgefäßes (4) in das Misch-Absetzgefäß (2) gibt, die darin erhaltene Unterphase in das Misch-Absetzgefäß (1) gibt, die verbleibende obere Produktphase (2) aus dem Misch-Absetzgefäß (2) zusammen mit der Unterphase aus einem Misch-Absetzgefäß (5) und der Oberphase aus dem Misch-Absetzgefäß (4) in ein Misch-Absetzgefäß (3) gibt, die im Misch-Absetzgefäß (3) erhaltene Oberphase zusammen mit der für die Neutralisation erforderlichen Menge an Alkalihydroxid in das Misch-Absetzgefäß (5) gibt, die im Misch-Absetzgefäß (3) erhaltene Unterphase gleichzeitig mit einer solchen Menge der Produktphase (2) in das Misch-Absetzgefäß (4) gibt, daß dort ein pH-Wert von 0,5 bis 7 aufrechterhalten wird, die im Absetzgefäß (4) erhaltene Unterphase in das Misch-Absetzgefäß (2) gibt, die im Misch-Absetzgefäß (5) erhaltene Unterphase zusammen mit der Produktphase (2) in das Misch-Absetzgefäß (3) gibt, die im Misch-Absetzgefäß (5) erhaltene Oberphase durch Zugabe einer Teilmenge der Produktphase (2) auf einen pH-Wert von 3 bis 9 einstellt und durch Eindampfen aufkonzentriert.

Dieses Verfahren soll anhand der anliegenden Schemazeichnung näher erläutert werden.

Die von $SO_2$ befreite Reaktionsmischung aus der Sulfoxidation mit der umgefähren Zusammensetzung:

39-41 % $H_2O$
7-8 % $H_2SO_4$
20-23 % $RSO_3H$ R = $C_{13}$-$C_{17}$ n-Paraffingemisch
30-32 % Paraffin

wird in einen Mixer-Settler 1 geführt, in den gleichzeitig die Unterphase aus einem Mixer-Settler 2, bestehend aus einer ca. 8-14 %-igen Schwefelsäure mit ca. 2-7 % Natriumhydrogensulfat, läuft.

Gleichzeitig wird in den Mixer 1 soviel wassergesättigtes Alkanol (in Abhängigkeit von der C-Kettenlänge des verwendeten Alkanols 15-40 %, im Falle des bevorzugten Isobutanols ca. 18-40 %, insbesondere 21-30 %, bezogen auf die Menge des entgasten Extraktes) hineingepumpt, daß im Settler 1 sich eine ca. 15-20 %ige Schwefelsäure als Unterphase abtrennt, die höchstens mit geringen Mengen an Alkalihydrogensulfat (z.B. <1,5 % $NaHSO_4$, bezogen auf die eingedampfte ca. 80 %ige Schwefelsäure) verunreinigt ist und ohne weiteres auf 78-80 % eingedampft werden kann, ohne daß nachträglich Alkalihydrogensulfat auskristallisiert.

Die Menge des an dieser Stelle zugesetzten Alkanols beeinflußt den Alkalisulfatgehalt im Endprodukt, d.h. in der Paraffinsulfatschmelze, und zwar so, daß der Restsalzgehalt mit zunehmender Alkanolmenge abnimmt. Ein über 35 % hinausgehender Alkanolanteil führt jedoch nur noch zu einer unwesentlichen Abnahme dieses Gehaltes in der Schmelze.

Die Oberphase aus dem Settler 1 wird im Mixer 2 mit der Unterphase aus einem Settler 4, die bei Verwendung von Isobutanol als Alkanol im wesentlichen aus einer ca. 3-10 %igen wäßrigen Alkalihydrogensulfatlösung besteht, verrührt. Die darauf im Settler 2 abgetrennte Oberphase fließt in ein Puffergefäß, aus dem jeweils soviel alkanolische und paraffinhaltige Alkansulfonsäurelösung in den Mixer 4 gefördert wird, daß sich dort ein pH-Wert zwischen 0,5 und 7, vorzugsweise 1-5, insbesondere jedoch 1-1,5 einstellt.

Die übrige Alkansulfonsäurelösung, die ungefähr 2/3 des gesamten Mengenstromes ausmacht, der durch das Puffergefäß fließt, wird in einem Mixer 3 mit der Oberphase aus dem Settler 4, bestehend aus sauer reagierender, alkanolischer Alkansulfonatlösung und mit der Unterphase aus einem Settler 5, bestehend aus einer wäßrigen Alkali/Alkalisulfatlösung (z. B. ca. 1-5 % $Na_2SO_4$ und ca. 10-40 % NaOH im Falle der bevorzugten Isobutanols) gemischt.

Die Unterphase des Settlers 3, ebenfalls eine alkalische Alkalisulfatlösung (die z.B. 6-10 % $Na_2SO_4$ und 0,5-8 % NaOH enthalten kann) läuft in den Mixer 4, während die Oberphase des Settlers 3, eine alkalisch reagierende Alkansulfonatlösung, im Mixer 5 mit der für die Neutralisation der gesamten Alkansulfonsäure notwendigen Natronlaugemenge versetzt wird.

Die alkalisch reagierende Oberphase des Settlers 5 fließt in die Neutralisation, wo sie mit einem Teil der Sulfonsäurelösung aus dem Puffergefäß auf einen pH-Wert von 3 bis 9, vorzugsweise 5 bis 8, eingestellt wird.

Dieses Neutralisat wird schließlich in zwei hintereinander geschalteten Fallfilmverdampfern zur Schmelze eingedampft. Im ersten Fallfilmdampfer (Heizmanteldampfdruck ca. 3-6 bar) destilliert der größte Teil des Wassers und Isobutanols bei Normaldruck ab. Im zweiten Fallfilmverdampfer, der bei einem Heizmanteldampfdurck von 20-30 bar, insbesondere bei ca. 22-26 bar im Vakuum von 15-30 mbar, insbesondere 17-19 mbar betrieben wird, verdampft das Paraffin zusammen mit den restlichen Wasser- und Isobutanolmengen.

Die so erhaltene Paraffinsulfonat-Schmelze besteht zu ca. 97-98 % aus Alkansulfonat, zu 0.8-1.3 % aus Paraffin und zu 1,0-1,7 % aus $Na_2SO_4$.

Die Mixer-Settler 1 und 2 werden bei Temperaturen von 12 bis 80 °C, vorzugsweise 25-50 °C, insbesondere 30-35 °C betrieben. Die Mixer-Settler 3 und 5 sollten zwecks optimaler Phasentrennung möglichst nahe am Siedepunkt des Gemisches, z.B. im Falle des Isobutanols als Alkanol bei Normaldruck bei 88-91 °C betrieben werden. Nicht mehr ganz so gut, aber immer noch hinreichend gut und mit steigender Temperatur besser werdend, läuft mit Isobutanol als Alkanol die Phasentrennung auch bei 40-88 °C ausreichend schnell und vollständig ab. Der Mixer Settler 4 kann dann bei Temperaturen von 40-80 °C, aber auch bei Temperaturen bis zu 91 °C betrieben werden, wobei eine Temperatur von 60-85 °C, insbesondere 70 °C optimal ist.

In analoger Weise können natürlich auch die höheren $C_5$-$C_8$-Alkanole eingesetzt werden, die den Vorteil haben, daß mit wachsender C-Kettenlänge des Alkanols der Restsulfatgehalt im Endprodukt geringfügig weiter herabgesetzt werden kann. Von Nachteil ist hierbei jedoch, daß der Aufwand an Energie zur Trennung von Wasser und Alkanol auf der einen und Alkanol und Paraffin auf der anderen Seite sehr schnell anwächst.

Als Alkalisierungsmittel eignen sich neben NaOH auch Kalilauge oder andere Basen wie z.B. MgO oder $Mg(OH)_2$, ZnO oder Gemische aus diesen.

Die Vorteile dieses Verfahrens sind darin zu sehen, daß kein Alkalisulfat als Abfallprodukt anfällt, sondern nur Schwefelsäure. Dadurch gehen weniger Alkali-Ionen im gesamten Prozeß verloren. Dies hat zur Folge, daß sich die Menge an Alkalihydroxid verringert, die zur Neutralisation benötigt wird. Von Vorteil ist außerdem, daß hier im Vergleich zu dem vorbekannten Verfahren der Gehalt an Alkoholen in dem abdestillierten Abwasser sehr viel geringer ist.

Beispiel

27 kg eines Sulfoxidations-Reaktionsgemischs der folgenden Zusammensetzung
40 % Wasser
7,5 % Schwefelsäure
21,5 % $RSO_3H$ R = $C_{13}$-$C_{17}$-Alkyl
31 % Paraffin
werden in einen Mixer-Settler 1 gegeben, in den gleichzeitig die Unterphase aus einem Mixer-Settler 2, bestehend aus 10 %iger Schwefelsäure mit einem Gehalt von ca. 4 % Natriumhydrogensulfat läuft. Gleichzeitig wird in den Mixer 1 23 %, bezogenen auf die Menge an Sulfoxidation-Reaktionsgemisch, mit Wasser gesättigtes Isobutanol hineingepumpt. Dabei scheidet sich im Settler 1 eine 17 %ige Schwefelsäure als Unterphase ab, die noch ca. 1 % Natriumhydrogensulfat, bezogen auf eingedampfte 80 %ige Schwefelsäure, enthält. Diese Schwefelsäure wird aus dem gesamten Kreislauf abgetrennt und durch Eindampfen aufkonzentriert.

Die Oberphase aus dem Settler 1 wird im Mixer 2 mit der Unterphase aus einem Settler 4, die im wesentlichen aus einer ca. 6 %igen Natriumhydrogensulfat-Lösung besteht, verrührt. Die daraufin im Settler 2 abgetrennte Oberphase fließt dann in ein Puffergefäß, aus dem jeweils soviel in den Mixer 4 gefördert wird, daß sich dort ein pH-Wert von 1 einstellt. Die übrige Alkansulfonsäure-Lösung aus dem Puffergefäß, die ungefähr 2/3 des gesamten Mengenstroms ausmacht, der durch das Puffergefäß fließt, wird in einem Mixer 3 mit der Oberphase aus dem Settler 4, bestehend aus sauer reagierender, isobutanolischer Alkansulfonat-Lösung und mit der Unterphase aus dem Settler 5, bestehend aus einer wäßrigen Lösung mit einem Gehalt von 14 % NaOH und 3,5 % $Na_2SO_4$, gemischt.

Die Unterphase des Settlers 3, ebenfalls eine alkalische Natriumsulfat-Lösung mit einem Gehalt von 2,7 % NaOH und 8 % $Na_2SO_4$, läuft in den Mixer 4, während die Oberphase des Settlers 3, eine alkalisch reagierende Alkansulfonat-Lösung im Mixer 5 mit der für die Neutralisation der gesamten Alkansulfonsäure notwendigen Menge an Natronlauge versetzt wird. Die Menge beträgt ca. 2,0 kg NaOH (50 %ig). Die alkalisch reagierende Oberphase des Settlers 5 fließt in die Neutralisationsstufe, wo sie mit einem Teil der Sulfonsäurelösung aus dem Puffergefäß auf den pH-Wert 7 eingestellt wird.

Dieses Neutralisat wird schließlich in zwei hintereinandergeschalteten Fallfilmverdampfen zur Schmelze eingedampft. Im ersten Fallfilmverdampfer (Heizmanteldampfdruck ca. 4 bar) destilliert der größte Teil des Wassers und des Isobutanols bei Normaldruck ab. Im zweiten Fallfilmverdampfer , der bei einem Heizmanteldampfdruck von 24 bar im Vakuum von 18 mbar betrieben wird, verdampft das Paraffin zusammen mit restlichen Mengen an Wasser und Isobutanol.

Die so erhaltene Schmelze des Alkansulfonats besteht zu 97,5 % aus Na-Alkansulfonat, 1 % Paraffin und 1,5 % Natriumsulfat.

Die Mixer-Settler 1 und 2 werden bei 34 °C betrieben, die Mixer-Settler 3 und 5 bei 90 °C und der Mixer-Settler 4 bei 75 °C.

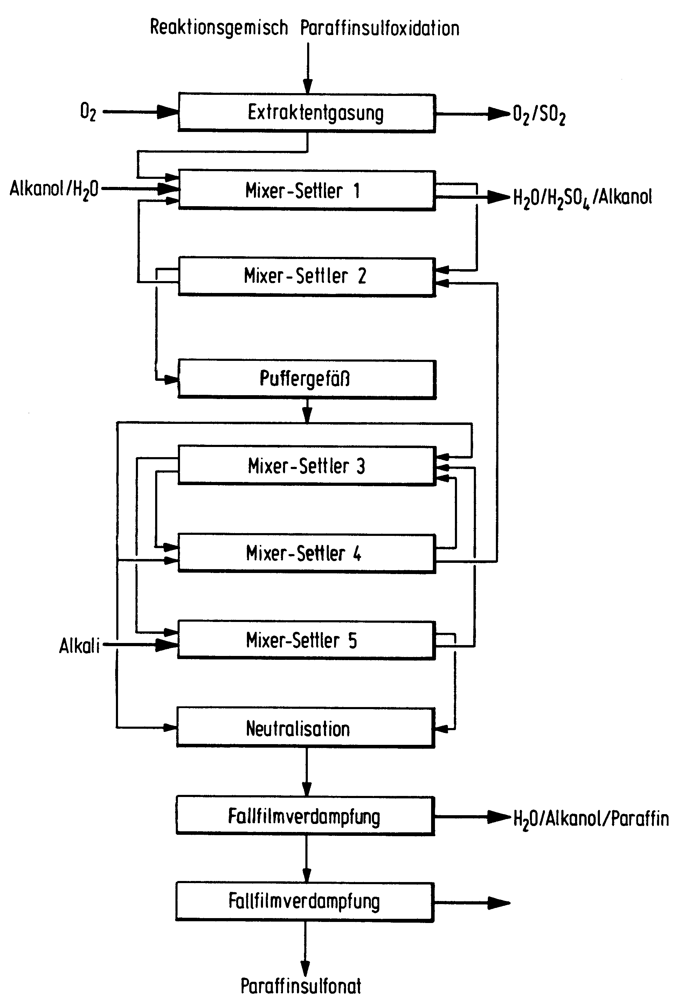

## Patentansprüche

1. Verfahren zur Isolierung von Alkalisulfat-armen Alkansulfonaten und Schwefelsäure aus Paraffin-Sulfoxidation-Reaktionsgemischen mit Hilfe von Alkoholen, dadurch gekennzeichnet, daß man das von Schwefeldioxid befreite Reaktionsgemisch in einem Misch-Absetzgefäß (1) mit einem $C_4$-$C_8$-Alkohol und der Unterphase aus einem Misch-Absetzgefäß (2) vermischt, die sich abscheidende untere Phase von verdünnter Schwefelsäure abtrennt, die verbleibende obere Produktphase zusammen mit der Unterphase eines Misch-Absetzgefäßes (4) in das Misch-Absetzgefäß (2) gibt, die darin erhaltene Unterphase in das Misch-Absetzgefäß (1) gibt, die verbleibende obere Produktphase aus dem Misch-Absetzgefäß (2) zusammen mit der Unterphase aus einem Misch-Absetzgefäß (5) und der Oberphase aus dem Misch-Absetzgefäß (4) in ein Misch-Absetzgefäß (3) gibt, die im Misch-Absetzgefäß (3) erhaltene Oberphase zusammen mit der für die Neutralisation erforderlichen Menge an Alkalihydroxid in das Misch-Absetzgefäß (5) gibt, die im Misch-Absetzgefäß (3) erhaltene Unterphase gleichzeitig mit einer solchen Menge der Produktphase (2) in das Misch-Absetzgefäß (4) gibt, daß dort ein pH-Wert von 0,5 bis 7 aufrechterhalten wird, die im Absetzgefäß (4) erhaltene Unterphase in das Misch-Absetzgefäß (2) gibt, die im Misch-Absetzgefäß (5) erhaltene Unterphase zusammen mit der Produktphase (2) in das Misch-Absetzgefäß (3) gibt, die im Misch-Absetzgefäß (5) erhaltene Oberphase durch Zugabe einer Teilmenge der Produktphase (2) auf einen pH-Wert von 3 bis 9 einstellt und durch Eindampfen aufkonzentriert.

2. Verfahren nach Anspruch 1, dadurch gekennzeichnet, daß man in dem Misch-Absetzgefäß-(4)einen pH-Wert von 1 bis 5 aufrecht erhält.

## Claims

1. A process for isolating alkanesulfonates having a low alkali metal sulfate content and sulfuric acid from paraffin-sulfoxidation reaction mixtures with the aid of alcohols, wherein the reaction mixture, freed from sulfur dioxide, is mixed in a mixer/settler vessel (1) with a $C_4$-$C_8$-alcohol and the lower phase from a mixer/settler vessel (2), the dilute sulfuric acid lower phase which separates out is removed, the upper product phase remaining is transferred, together with the lower phase from a mixer/settler vessel (4), into mixer/settler vessel (2), the lower phase obtained in the latter is transferred into mixer/settler vessel (1), the remaining upper product phase from mixer/settler vessel (2), together with the lower phase from a mixer/settler vessel (5) and the upper phase from mixer/settler vessel (4) are transferred into a mixer/settler vessel (3), the upper phase obtained in mixer/settler vessel (3), together with the amount of alkali metal hydroxide which is necessary for neutralization, is transferred into mixer/settler vessel (5), the lower phase obtained in mixer/settler vessel (3) is transferred into mixer/settler vessel (4) at the same time as an amount of the product phase (2) such that a pH of 0.5 to 7 is maintained in the latter vessel, the lower phase obtained in the settler vessel (4) is transferred into mixer/settler vessel (2), the lower phase obtained in mixer/settler vessel (5), together with the product phase (2), is transferred into mixer/settler vessel (3), and the upper phase obtained in mixer/settler vessel (5) is adjusted to a pH of 3 to 9 by adding part of the product phase (2) and concentrated by evaporation.

2. The process as claimed in claim 1, wherein a pH of 1 to 5 is maintained in mixer/settler vessel (4).

**Revendications**

1. Procédé pour isoler des alcanesulfonates à faible teneur en sulfates de métaux alcalins, ainsi que de l'acide sulfurique, à partir de mélanges réactionnels de sulfoxydation de paraffines, à l'aide d'alcools, caractérisé en ce qu'on mélange le mélange réactionnel débarrassé de l'anhydride sulfureux, dans un mélangeur-séparateur (1), avec un alcool en $C_4$-$C_8$ et la phase inférieure d'un mélangeur-séparateur (2), on sépare de l'acide sulfurique dilué la phase inférieure qui se dépose, on introduit dans le mélangeur-séparateur (2) la phase produit supérieure restante, avec la phase inférieure d'un mélangeur-séparateur (4), on introduit la phase inférieure qui y est obtenue dans le mélangeur-séparateur (1), on introduit dans un mélangeur-séparateur (3) la phase produit supérieure restante (2) du mélangeur-séparateur (2), en même temps que la phase inférieure du mélangeur-séparateur (5) et la phase supérieure du mélangeur-séparateur (4), on introduit dans le mélangeur-séparateur (5) la phase supérieure obtenue dans le mélangeur-séparateur (3), en même temps que la quantité d'hydroxyde de métal alcalin nécessaire à la neutralisation, on introduit dans le mélangeur-séparateur (4) la phase inférieure obtenue dans le mélangeur-séparateur (3), en même temps qu'une quantité de la phase produit (2) telle que l'on y maintienne un pH de 0,5 à 7, on introduit dans le mélangeur-séparateur (2) la phase inférieure obtenue dans le séparateur (4), on introduit dans le mélangeur-séparateur (3) la phase inférieure obtenue dans le mélangeur-séparateur (5) en même temps que la phase produit (2), on ajuste à une valeur de 3 à 9 le pH de la phase supérieure obtenue dans le mélangeur-séparateur (5), par addition d'une quantité partielle de la phase produit (2), et on concentre par évaporation.

2. Procédé selon la revendication 1, caractérisé en ce qu'on maintient dans le mélangeur-séparateur (4) un pH de 1 à 5.

Reaktionsgemisch Paraffinsulfoxidation

$O_2$ → | Extraktentgasung | → $O_2/SO_2$

Alkanol/$H_2O$ → | Mixer-Settler 1 | → $H_2O/H_2SO_4$/Alkanol

| Mixer-Settler 2 |

| Puffergefäß |

| Mixer-Settler 3 |

| Mixer-Settler 4 |

Alkali → | Mixer-Settler 5 |

| Neutralisation |

| Fallfilmverdampfung | → $H_2O$/Alkanol/Paraffin

| Fallfilmverdampfung | →

Paraffinsulfonat

7